# EUROPEAN PATENT APPLICATION

(11) **EP 4 628 047 A1**
(43) Date of publication of application: **08.10.2025**
(21) Application number: 23898110.4
(22) Date of filing: 02.11.2023
(51) Int. Cl.: A61C 7/00, A61C 9/00, G06T 17/00, G06T 19/20, G16H 30/00, G16H 50/50

(54) **METHOD AND DEVICE FOR PROVIDING PROBLEM SOLVING INFORMATION RELATED TO ORTHODONTICS**

(30) Priority: 01.12.2022 KR 20220165670
(71) Applicant: Osstem Implant Co., Ltd., Gangseo-gu Seoul 07789 (KR)
(72) Inventor: CHOI, Kyoo Ok, Seoul 07789 (KR); KIM, Hwa Sam, Gimpo-si Gyeonggi-do 10113 (KR)
(74) Representative: Klunker IP Patentanwälte PartG mbB
(86) International application number: PCT/KR2023/017429
(87) International publication number: WO 2024/117578

(57) **Abstract**

According to an embodiment, disclosed are a method, a device implementing the method, and a recording medium, the method comprising the steps of: obtaining, by a reception module, input information including information about a current tooth position; obtaining, by a processor, a plurality of expected orthodontic models respectively representing expected tooth positions that are updated as an orthodontic treatment is carried out according to a treatment goal; obtaining, by the processor, a problem solving table including a plurality of problem items and a problem solving method corresponding to each of the plurality of problem items and representing movement or rotation of teeth; on the basis of the problem solving table and a comparison result of a current tooth model representing the current tooth position with the plurality of expected orthodontic models, obtaining, by the processor, an item to be solved, which is a problem item to be solved among the plurality of problem items; and displaying, by a display, the item being solved and a solution situation graphic corresponding to the item being solved.

## Description

### [Technical Field]

The present disclosure relates to a device and method for providing problem solving information related to orthodontic treatment. Specifically, it relates to a technical field for displaying problem solving information in a concrete manner so that a user can more easily identify the status in which problem items are being solved during the course of orthodontic treatment.

### [Background Art]

In the course of orthodontic treatment, it is not the case that only one issue is resolved at a time; rather, it is common for multiple issues to be resolved simultaneously as the treatment progresses. As orthodontic treatment progresses, the positions of teeth are updated, and the resolution of various issues does not necessarily occur at the same time.

In particular, when resolving multiple complex problems simultaneously, there is a need for a method that enables easier identification of the problem solving status.

Accordingly, there is a need for a method capable of more concretely visualizing and providing which problem items are being solved and at what points in time they are solved as the orthodontic treatment progresses.

### [Disclosure]

### [Technical problem]

One embodiment of the present disclosure is intended to solve the problems of the prior art described above, and includes a technical problem of visualizing and displaying which problem item is being solved by using a current tooth position and an expected tooth position.

The technical problems to be solved are not limited to those described above, and may further include various technical problems that would be apparent to those of skilled in the art.

### [Technical Solution]

According to a first aspect of the present disclosure, a method for providing problem solving information related to orthodontic treatment by a device may include the steps of: obtaining, by a reception module, input information including information about a current tooth position; obtaining, by a processor, a plurality of expected orthodontic models, each representing in three dimensions an expected tooth position that is updated as orthodontic treatment progresses according to a treatment goal; obtaining, by the processor, a problem solving table including a plurality of problem items and problem solving methods respectively corresponding to the plurality of problem items and representing movement or rotation of teeth; on the basis of the problem solving table and a comparison result of a current tooth model representing the current tooth position with the plurality of expected orthodontic models, obtaining, by the processor, an item to be solved, which is a problem item to be solved among the plurality of problem items; and displaying, by a display, the item to be solved and a solution situation graphic corresponding to the item to be solved.

In addition, the problem solving table may represent movement of a tooth for solving the plurality of problem items as at least one of a first direction movement corresponding to an occlusal direction, a second direction movement corresponding to a buccal direction, or a third direction movement corresponding to a mesial direction, and may represent rotation of a tooth for solving the plurality of problem items as at least one of a first rotation about the first direction, a second rotation about the second direction, or a third rotation about the third direction.

In addition, the problem solving table may indicate, for each of a plurality of teeth, the movement or rotation of a tooth for solving the plurality of problem items, among the first direction movement, the second direction movement, the third direction movement, the first rotation, the second rotation, and the third rotation.

Additionally, the obtaining of the item to be solved may include the steps of: determining, on the basis of the comparison result, at least one movement or rotation to be performed during orthodontic treatment from among the first direction movement, the second direction movement, the third direction movement, the first rotation, the second rotation, and the third rotation; determining at least one problem item corresponding to the at least one determined movement or rotation based on the problem solving table; and determining the at least one determined problem item as the item to be solved.

In addition, the displaying of the solution situation graphic may include displaying a variation amount corresponding to the item to be solved together with the item to be solved.

Additionally, the plurality of expected orthodontic models may be obtained on the basis of expected tooth positions corresponding to a plurality of time points according to a preset time interval.

In addition, the solution situation graphic may represent, in the form of a bar, a time period within the overall orthodontic treatment period that corresponds to the item to be solved.

In addition, the displaying of the solution situation graphic may include displaying a time period corresponding to the item to be solved together with the item to be solved.

In addition, the plurality of problem items may be determined based on the current tooth position.

In addition, the obtaining of the plurality of expected orthodontic models may include the steps of: determining an expected item to be solved, which is a problem item expected to require resolution among the plurality of problem items; determining the item to be solved by updating the expected item to be solved based on user input; and obtaining the plurality of expected orthodontic models based on the item to be solved.

In addition, the comparison result may include a variation amount representing a positional difference or angular difference between a current tooth represented by the current tooth model and an expected tooth represented by the expected tooth orthodontic model.

According to a second aspect of the present disclosure, a device for providing problem solving information related to orthodontic treatment may include: a reception module configured to receive input information including information about a current tooth position; a processor configured to obtain a plurality of expected orthodontic models, each representing in three dimensions an expected tooth position that is updated as orthodontic treatment progresses according to a treatment goal, obtain a problem solving table including a plurality of problem items and problem solving methods respectively corresponding to the plurality of problem items and representing movement or rotation of teeth, and on the basis of the problem solving table and a comparison result of a current tooth model representing the current tooth position with the plurality of expected orthodontic models, obtain an item to be solved, which is a problem item to be solved among the plurality of problem items; and a display configured to display the item to be solved and a solution situation graphic corresponding to the item to be solved.

Additionally, the problem solving table may represent movement of a tooth for solving the plurality of problem items as at least one of a first direction movement corresponding to an occlusal direction, a second direction movement corresponding to a buccal direction, or a third direction movement corresponding to a mesial direction, and may represent rotation of a tooth for solving the plurality of problem items as at least one of a first rotation about the first direction, a second rotation about the second direction, or a third rotation about the third direction.

In addition, the problem solving table may indicate, for each of a plurality of teeth, the movement or rotation of a tooth for solving the plurality of problem items, among the first direction movement, the second direction movement, the third direction movement, the first rotation, the second rotation, and the third rotation.

Additionally, the processor may be configured to determine, on the basis of the comparison result, at least one movement or rotation to be performed during orthodontic treatment from among the first direction movement, the second direction movement, the third direction movement, the first rotation, the second rotation, and the third rotation, determine at least one problem item corresponding to the at least one determined movement or rotation based on the problem solving table, and determine the at least one determined problem item as the item to be solved.

Additionally, the processor may be configured to determine an expected item to be solved, which is a problem item expected to require resolution among the plurality of problem items, determine the item to be solved by updating the expected item to be solved based on user input, and obtain the plurality of expected orthodontic models based on the item to be solved.

According to a third aspect of the present disclosure, there is provided a computer-readable recording medium having recorded thereon a program, which, when executed by a computer, performs the method of the first aspect.

Other specific details are included in the detailed description and drawings.

### [Effects of the Invention]

According to an embodiment, it is possible to visually display, using graphics or the like, which problem item is being solved based on the current tooth position and the expected tooth position. According to the present disclosure, since the user can intuitively identify which problem item is being solved during the orthodontic treatment process based on tooth movement or rotation, the device according to the present disclosure can be effectively utilized in establishing an orthodontic treatment plan.

It should be understood that the effects of the present disclosure are not limited to the aforementioned effects and include all effects that can be inferred from the configurations of the invention described in the detailed description or the claims.

### [Description of Drawings]

FIG. 1 is a block diagram schematically illustrating an example of a configuration of device according to an embodiment.
FIG. 2 is a flowchart illustrating respective steps performed by a device to display a solution situation graphic according to an embodiment.
FIG. 3 is a diagram for explaining directions with respect to a tooth according to an embodiment.
FIG. 4 is a table for explaining directions of movement or rotation of a tooth according to an embodiment.
FIG. 5 is a table for explaining a problem solving table including problem items and problem solving methods according to an embodiment.
FIG. 6 is a diagram for explaining an example of a solution situation graphic corresponding to an item to be solved according to an embodiment.

### [Mode of the Invention]

The terms used in the embodiments are those general terms currently widely used in the art in consideration of functions in regard to the inventive concept, but the terms may vary according to the intention of those of ordinary skill in the art, precedents, or new technology in the art. Also, specified terms may be selected by the applicant, and in this case, the detailed meaning thereof will be described in the detailed description of the inventive concept. Thus, the terms used in the specification should be understood not as simple names but based on the meaning of the terms and the overall description of the inventive concept.

Throughout the specification, it will also be understood that when a component "comprises," or "includes" an element, unless there is another opposite description thereto, it should be understood that the component does not exclude another element but may further include another element. Furthermore, the term "unit (or part)" used in the specification refers to a unit for processing at least one function or operation, and this may be realized in the form of hardware, software, or in a combination of both hardware and software.

Embodiments of the present invention will now be described in detail with reference to the accompanying drawings so that those skilled in the art can easily practice the embodiments of the present disclosure. However, the present disclosure may be implemented in many different forms and is not limited to the embodiments described herein.

Hereinafter, a plurality of embodiments of the present disclosure will be described in detail with reference to the drawings.

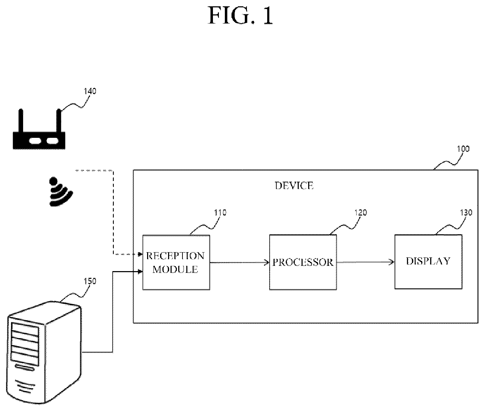

FIG. 1 is a block diagram schematically illustrating an example of a configuration of device 100 according to an embodiment.

Referring to FIG. 1, a device 100 may include a reception module 110, a processor 120, and a display 130.

It will be understood by those skilled in the art that, in addition to the components illustrated in FIG. 1, other general-purpose components may also be included in the device 100. For example, the device 100 may further include a memory (not shown). Alternatively, it will also be understood by those skilled in the art that, according to other embodiments, some components illustrated in FIG. 1 may be omitted.

According to an embodiment, the reception module 110 may obtain input information including information on a current tooth position. The reception module 110 may obtain the input information from various external devices 140 and 150. For example, the reception module 110 may obtain the input information from a wireless terminal 140 via wireless communication. In another example, the reception module 110 may obtain the input information from a wired terminal 140 via wired communication.

The external devices 140 and 150 may include various devices such as a computerized tomography (CT) device, an X-ray device, or an intraoral scanner, and the device 100 may determine the current tooth position using the obtained information.

According to an embodiment, the processor 120 may obtain a plurality of expected orthodontic models respectively representing expected tooth positions that are updated as an orthodontic treatment is carried out according to a treatment goal. Since the tooth position is updated as the orthodontic treatment progresses, the processor 120 may determine the updated expected tooth position and obtain the corresponding expected orthodontic model.

According to an embodiment, the processor 120 may obtain a problem solving table including a plurality of problem items and problem solving methods respectively corresponding to the plurality of problem items and representing movement or rotation of teeth. The problem solving table may be pre-stored in a memory (not shown). The problem solving table may provide, for each of the plurality of problem items, movement or rotation of teeth required to solve the corresponding problem item.

The processor 120 according to an embodiment may obtain an item to be solved, which is a problem item to be solved among the plurality of problem items. Specifically, the processor 120 may obtain the item to be solved on the basis of the problem solving table and a comparison result of a current tooth model representing a current tooth position with the plurality of expected orthodontic models.

According to an embodiment, the display 130 may display the item to be solved and a solution situation graphic corresponding to the item to be solved. The solution situation graphic may visually represent, in a bar form or the like, a situation in which a plurality of items to be solved are solved over time.

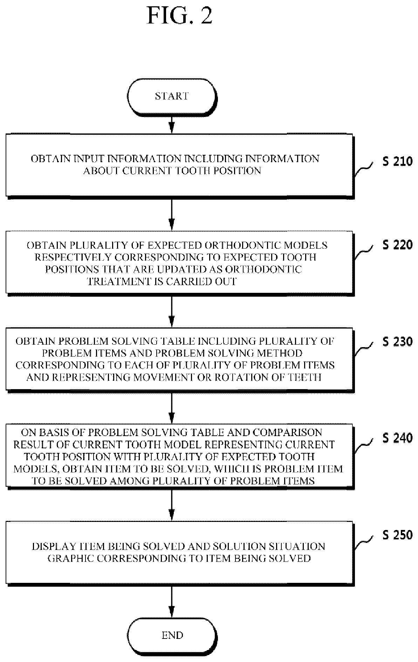

The embodiments described above with reference to FIG. 1 will now be described in more detail with reference to FIGS. 2 to 6.

FIG. 2 is a flowchart illustrating respective steps performed by the device 100 to display a solution situation graphic according to an embodiment.

In step S210, the device 100 according to an embodiment obtains input information including information on a current tooth position.

The device 100 according to an embodiment may obtain the input information from various types of sources. For example, the device 100 may obtain the input information from various devices such as a CT device, an X-ray device, or an intraoral scanner, and may determine the current tooth position using the obtained information. For example, the device 100 may determine the current tooth position using a CT image obtained from a CT device.

In step S220, the device 100 according to an embodiment obtains a plurality of expected orthodontic models respectively representing expected tooth positions that are updated as an orthodontic treatment is carried out according to a treatment goal.

Since the tooth position is updated as the orthodontic treatment progresses, the device 100 may determine the updated expected tooth position and obtain the corresponding expected orthodontic model.

Since the expected tooth position is continuously updated as the orthodontic treatment progresses, a plurality of expected tooth positions corresponding to respective time points may be obtained. For example, a third expected orthodontic model may be obtained for a third expected tooth position corresponding to a first period (e.g., one week) after the start of orthodontic treatment, and a fourth expected orthodontic model may be obtained for a fourth expected tooth position corresponding to a second period (e.g., fifteen days) after the start of orthodontic treatment.

According to an embodiment, the plurality of expected orthodontic models may be obtained on the basis of expected tooth positions corresponding to a plurality of time points according to a preset time interval. The preset time interval may be predetermined or may be determined based on user input or the like.

An example will now be described in which the preset time interval is one week according to an embodiment. The device 100 may obtain a first expected orthodontic model representing a first expected tooth position corresponding to a first time point, which is one week after the start of orthodontic treatment; a second expected orthodontic model representing a second expected tooth position corresponding to a second time point, which is two weeks after the start of orthodontic treatment; and an n-th expected orthodontic model representing an n-th expected tooth position corresponding to an n-th time point, which is n weeks after the start of orthodontic treatment.

According to an embodiment, the device 100 may obtain the expected orthodontic models as the item to be solved is determined.

The plurality of problem items may be determined based on the current tooth position, and the item to be solved may be an item among the plurality of problem items that is intended to be solved through orthodontic treatment. For example, among five problem items, the device 100 may determine that two are items to be solved through orthodontic treatment.

According to an embodiment, the device 100 may determine expected items to be solved, which are problem items expected to require resolution among the plurality of problem items. The expected items to be solved may include some or all of the plurality of problem items.

The device 100 may determine the item to be solved based on user input. For example, the device 100 may determine, as the items to be solved, five expected items to be solved that are selected based on user input, from among ten expected items to be solved.

According to an embodiment, the device 100 may obtain the plurality of expected orthodontic models based on the item to be solved. The device 100 may determine how the positions of a plurality of teeth should be updated to correspond to the item to be solved. In addition, the device 100 may obtain the plurality of expected orthodontic models corresponding to different time points over time.

In step S230, the device 100 according to an embodiment obtains a problem solving table including a plurality of problem items and problem solving methods respectively corresponding to the plurality of problem items and representing movement or rotation of teeth.

The problem solving table may represent movement of a tooth for solving the plurality of problem items as at least one of: a first direction movement corresponding to an occlusal direction, a second direction movement corresponding to a buccal direction, or a third direction movement corresponding to a mesial direction.

The problem solving table may represent rotation of a tooth for solving the plurality of problem items as at least one of: a first rotation about the first direction, a second rotation about the second direction, or a third rotation about the third direction.

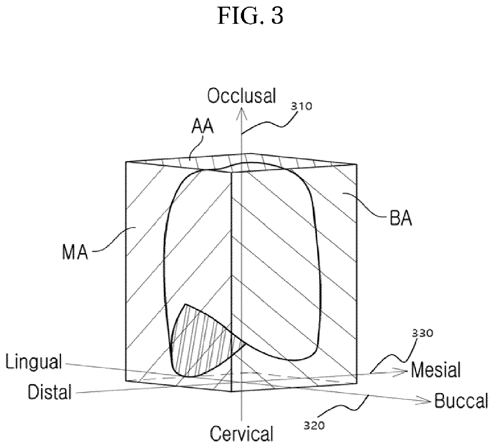

Specific examples for representing movement or rotation of a tooth will be described below with reference to FIGS. 2 and 3.

The problem solving table may be pre-stored, and may indicate, for each of a plurality of teeth, the movement or rotation of a tooth for solving the plurality of problem items, among the first direction movement, the second direction movement, the third direction movement, the first rotation, the second rotation, and the third rotation.

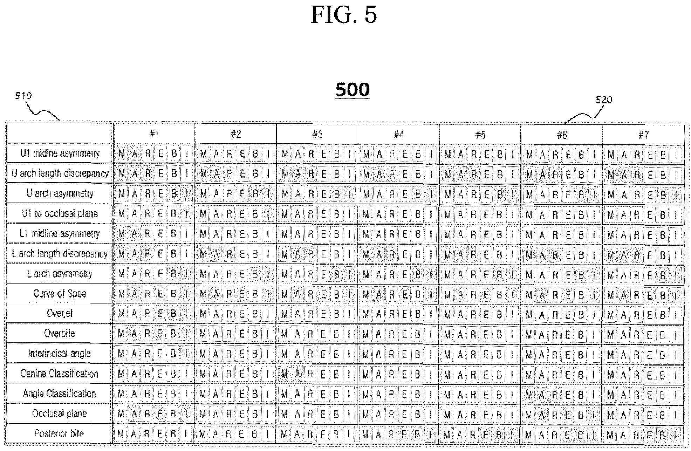

A specific example of the problem solving table will be described below with reference to FIG. 5.

In step S240, the device 100 according to an embodiment obtains an item to be solved, which is a problem item to be solved among the plurality of problem items.

According to an embodiment, the device 100 may obtain an item to be solved on the basis of the problem solving table and a comparison result of a current tooth model representing a current tooth position with the plurality of expected orthodontic models.

The comparison result according to an embodiment may include a variation amount indicating a positional difference or angular difference between the current tooth represented by the current tooth model and the expected tooth represented by the expected orthodontic model.

As the orthodontic treatment progresses, a positional difference or angular difference may occur between the current tooth represented by the current tooth model and the expected tooth represented by the expected orthodontic model. The device 100 may determine, on the basis of the variation amount (e.g., positional difference, angular difference, etc.) between the current tooth and the expected tooth, an item to be solved through orthodontic treatment among the plurality of problem items.

According to an embodiment, the device 100 may obtain the current tooth model representing the current tooth position. In addition, according to an embodiment, the device 100 may obtain a plurality of expected orthodontic models indicating how the positions of a plurality of teeth should be updated to correspond to the item to be solved. The device 100 may determine the item to be solved, which is a problem item to be solved among the plurality of problem items, on the basis of the comparison result of the current tooth model with the plurality of expected orthodontic models.

According to an embodiment, the device 100 may obtain the plurality of expected orthodontic models based on the item to be solved. The device 100 may determine how the positions of a plurality of teeth should be updated to correspond to the item to be solved. In addition, the device 100 may obtain the plurality of expected orthodontic models corresponding to different time points over time.

According to an embodiment, the device 100 may determine, on the basis of the comparison result, one or more movements or rotations to be performed during orthodontic treatment from among a first direction movement, a second direction movement, a third direction movement, a first rotation, a second rotation, and a third rotation. The first direction movement, the second direction movement, the third direction movement, the first rotation, the second rotation, and the third rotation may be expressed as positive or negative numerical values.

According to an embodiment, the device 100 may determine, on the basis of the problem solving table, one or more problem items corresponding to the determined one or more movements or rotations, and may determine the one or more determined problem items as the items to be solved. The problem solving table includes information on movement or rotation of teeth corresponding to each problem item. Accordingly, the device 100 may compare the one or more movements or rotations performed during orthodontic treatment with the pre-stored problem solving table and determine one or more problem items as the items to be solved.

According to another embodiment, the device 100 may determine the item to be solved based on user input. The item to be solved may be determined from among the plurality of problem items based on the user input. Once the item to be solved is determined, an expected orthodontic model may be obtained based on the item to be solved.

In step S250, the device 100 according to an embodiment displays the item to be solved and a solution situation graphic corresponding to the item to be solved.

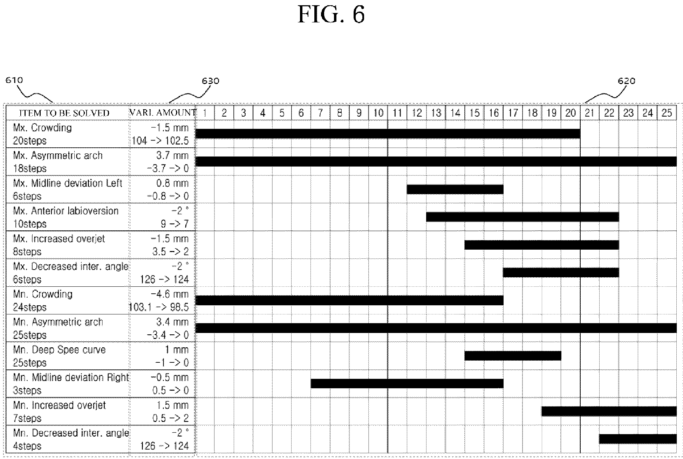

The solution situation graphic may visually represent a situation in which a plurality of items to be solved are solved over time. For example, the solution situation graphic may provide information, in units of a preset period (e.g., one week), as to whether each of the items to be solved is in the process of being solved or has been resolved as time progresses. A more specific example of the solution situation graphic will be described below with reference to FIG. 6.

According to an embodiment, the device 100 may determine, on the basis of a comparison result of the current tooth model with the plurality of expected orthodontic models, an item to be solved, which is a problem item to be solved among the plurality of problem items. In addition, when the device 100 provides the item to be solved in a visualized manner, such as through the solution situation graphic, the user may more easily identify the problem item and the item to be solved associated with the movement or rotation of teeth. Accordingly, the user may apply an appropriate modification input with reference to the visually provided information such as the solution situation graphic, and the device 100 may update the treatment goal or orthodontic method based on the received modification input. In this case, the expected orthodontic models may be updated, and the item to be solved may also be updated based on the updated expected orthodontic models.

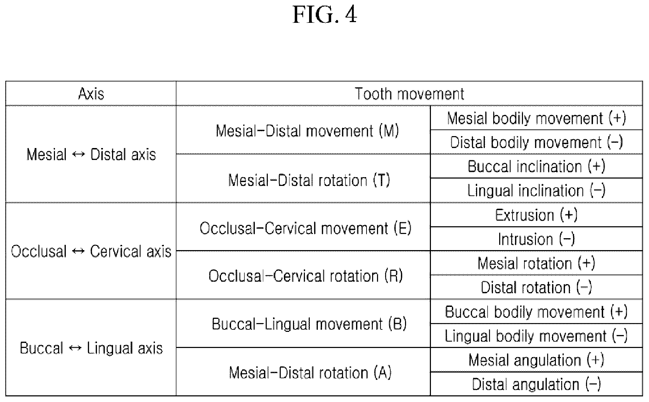

FIG. 3 is a diagram for explaining directions with respect to a tooth according to an embodiment, and FIG. 4 is a table for explaining directions of movement or rotation of a tooth according to an embodiment.

According to an embodiment, a first direction 310 with respect to a tooth may correspond to an occlusal direction, a second direction 320 may correspond to a buccal direction, and a third direction 330 may correspond to a mesial direction. In addition, referring to FIG. 3, the occlusocervical direction may correspond to the occlusal direction, the buccolingual direction may correspond to the buccal direction, and the mesiodistal direction may correspond to the mesial direction.

Accordingly, movement of a tooth may be represented as a positive or negative numerical value based on the first direction 310, the second direction 320, and the third direction 330. For example, when a tooth is moved in the first direction 310, the movement may be represented by a positive value with respect to the first direction 310, and when the tooth is moved in the opposite direction of the second direction 320, the movement may be represented by a negative value with respect to the second direction 320.

Rotation of a tooth may also be represented as a positive or negative numerical value based on the first direction 310, the second direction 320, and the third direction 330. For example, when the tooth is rotated in the buccal direction about the first direction 310 as an axis, the rotation may be represented by a positive value with respect to the first direction 310, and when the tooth is rotated in the distal direction about the second direction 320 as an axis, the rotation may be represented by a negative value with respect to the second direction 320.

Specifically, referring to FIG. 4, it can be confirmed that a total of six possible cases of movement and rotation are defined based on the first direction 310, the second direction 320, and the third direction 330.

FIG. 5 is a table for explaining a problem solving table 500 including problem items 510 and problem solving methods 520 according to an embodiment.

According to an embodiment, a problem solving table 500 may include problem items 510 and problem solving methods 520. The problem items 510 may represent issues that may arise in relation to orthodontic treatment, and the problem solving methods 520 may represent respective methods for solving each of the problem items 510. Specifically, the problem solving method 520 may indicate, among six possible cases representing three directions of movement of a tooth and three directions of rotation of a tooth, which direction of movement or rotation is required to solve each problem item 510.

For example, referring to FIG. 4, movement of a tooth along the first direction 310 may be represented as M, rotation of a tooth about the first direction 310 may be represented as T, movement of a tooth along the second direction 320 may be represented as E, rotation of a tooth about the second direction 320 may be represented as R, movement of a tooth along the third direction 330 may be represented as B, and rotation of a tooth about the third direction 330 may be represented as A. According to an embodiment, the problem solving table 500 may represent the problem solving method 520 for solving each problem item 510 by shading one or more possible cases that are required to be applied among six possible cases.

According to an embodiment, the problem solving table 500 may provide information on one or more teeth relevant to each problem item 510. In addition, the problem solving table 500 may indicate, for each problem item 510, movement or rotation of the relevant teeth with respect to at least one of the first direction 310, the second direction 320, and the third direction 330.

FIG. 6 is a diagram for explaining an example of a solution situation graphic 620 corresponding to an item 610 to be solved according to an embodiment.

Referring to FIG. 6, the device 100 may visually display a solution situation graphic 620 corresponding to each item 610 to be solved. For example, the solution situation graphic 620 may represent, in the form of a bar, whether each item 610 to be solved is in the process of being solved or has been completed, over time in units of a preset period (e.g., one week). For instance, the solution situation graphic 620 may represent, in the form of a bar, the time period within the overall orthodontic treatment period that corresponds to each item 610 to be solved.

According to an embodiment, the device 100 may display a variation amount 630 corresponding to the item 610 to be solved. For example, a variation amount 630 indicating that a tooth movement of approximately 3.7 mm is required to solve the problem item 610 labeled "Mx. Asymmetric arch" may be displayed.

In addition, the device 100 may display the time period corresponding to each item 610 to be solved. For example, information indicating that 20 unit periods (e.g., 20 weeks) are required to solve the problem item 610 labeled "Mx. Crowding" may be displayed as the phrase "20 steps."

According to an embodiment, the order in which the items 610 to be solved are displayed may be determined based on various factors. For example, the order of the items 610 to be solved may be determined based on factors such as the position of the tooth corresponding to the item to be solved, the resolution start time, the resolution end time, the duration required for resolution, and the variation amount.

According to an embodiment, the device 100 may determine the display order of the items 610 to be solved based on weights assigned in descending order to the following factors: the position of the tooth corresponding to the item 610 to be solved, the resolution start time, the resolution end time, the duration required for resolution, and the variation amount. When the order of the items 610 to be solved is determined in this manner, the solution situation may be presented in a way that facilitates visual recognition, as the solution situation graphic 620 is displayed not only with the upper jaw positioned at the top and the lower jaw at the bottom, but also in an order reflecting the resolution start time, the resolution end time, and the like. For example, an item to be solved corresponding to the upper jaw may be placed higher than an item to be solved corresponding to the lower jaw. An item to be solved with an earlier resolution start time may be placed higher than one with a later resolution start time. An item to be solved with an earlier resolution end time may be placed higher than one with a later resolution end time. An item to be solved requiring a longer resolution period may be placed higher than one requiring a shorter resolution period. An item to be solved with a larger variation amount may be placed higher than one with a smaller variation amount. The above-described embodiment is merely an example and should not be construed as limiting.

It should be appreciated that the order and combination of the steps shown above is merely an embodiment of the present disclosure, and the order, combination, branch, function and the performing subject may vary to be implemented with addition, fewer, or different steps without departing from the essential characteristics of each component described in the specification. Throughout this specification, the term "provide (or providing)" may be interpreted as comprehensively including a process in which an object obtains specific information or directly or indirectly transmits or receives specific information to or from a specific object and including the performance of related operations required in this process.

Various embodiments set forth herein may be implemented as software comprising one or more instructions stored in a storage medium (e.g., memory) that is readable by a machine (e.g., a display device or a computer). For example, a processor (e.g., the processor 120) of the machine may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

It will be understood by one of ordinary skill in the art that various changes in form and details may be made therein without departing from the spirit and scope of the exemplary embodiments as defined by the following claims. Thus, the disclosed methods should be considered in a descriptive sense only and not for purposes of limitation. The scope of the present disclosure is presented not in the foregoing description but in the claims and all differences within an equivalent range thereto should be construed as being included in the present disclosure.

## Claims

1. A method for providing problem solving information related to orthodontic treatment by a device, the method comprising the steps of:
obtaining, by a reception module, input information including information about a current tooth position;
obtaining, by a processor, a plurality of expected orthodontic models, each representing in three dimensions an expected tooth position that is updated as orthodontic treatment progresses according to a treatment goal;
obtaining, by the processor, a problem solving table including a plurality of problem items and problem solving methods respectively corresponding to the plurality of problem items and representing movement or rotation of teeth;
on the basis of the problem solving table and a comparison result of a current tooth model representing the current tooth position with the plurality of expected orthodontic models, obtaining, by the processor, an item to be solved, which is a problem item to be solved among the plurality of problem items; and
displaying, by a display, the item to be solved and a solution situation graphic corresponding to the item to be solved.

2. The method of claim 1, wherein the problem solving table represents movement of a tooth for solving the plurality of problem items as at least one of a first direction movement corresponding to an occlusal direction, a second direction movement corresponding to a buccal direction, or a third direction movement corresponding to a mesial direction, and represents rotation of a tooth for solving the plurality of problem items as at least one of a first rotation about the first direction, a second rotation about the second direction, or a third rotation about the third direction.

3. The method of claim 2, wherein the problem solving table indicates, for each of a plurality of teeth, the movement or rotation of a tooth for solving the plurality of problem items, among the first direction movement, the second direction movement, the third direction movement, the first rotation, the second rotation, and the third rotation.

4. The method of claim 2, wherein the obtaining of the item to be solved comprises the steps of:
determining, on the basis of the comparison result, at least one movement or rotation to be performed during orthodontic treatment from among the first direction movement, the second direction movement, the third direction movement, the first rotation, the second rotation, and the third rotation;
determining at least one problem item corresponding to the at least one determined movement or rotation based on the problem solving table; and
determining the at least one determined problem item as the item to be solved.

5. The method of claim 1, wherein the displaying of the solution situation graphic comprises displaying a variation amount corresponding to the item to be solved together with the item to be solved.

6. The method of claim 1, wherein the plurality of expected orthodontic models are obtained on the basis of expected tooth positions corresponding to a plurality of time points according to a preset time interval.

7. The method of claim 1, wherein the solution situation graphic represents, in a form of a bar, a time period within an overall orthodontic treatment period that corresponds to the item to be solved.

8. The method of claim 1, wherein the displaying of the solution situation graphic comprises displaying a time period corresponding to the item to be solved together with the item to be solved.

9. The method of claim 1, wherein the plurality of problem items are determined based on the current tooth position.

10. The method of claim 1, wherein the obtaining of the plurality of expected orthodontic models comprises the steps of:
determining an expected item to be solved, which is a problem item expected to require resolution among the plurality of problem items;
determining the item to be solved by updating the expected item to be solved based on user input; and
obtaining the plurality of expected orthodontic models based on the item to be solved.

11. The method of claim 1, wherein the comparison result comprises a variation amount representing a positional difference or angular difference between a current tooth represented by the current tooth model and an expected tooth represented by the expected tooth orthodontic model.

12. A device for providing problem solving information related to orthodontic treatment, comprising:
a reception module configured to receive input information including information about a current tooth position;
a processor configured to
obtain a plurality of expected orthodontic models, each representing in three dimensions an expected tooth position that is updated as orthodontic treatment progresses according to a treatment goal,
obtain a problem solving table including a plurality of problem items and problem solving methods respectively corresponding to the plurality of problem items and representing movement or rotation of teeth, and
on the basis of the problem solving table and a comparison result of a current tooth model representing the current tooth position with the plurality of expected orthodontic models, obtain an item to be solved, which is a problem item to be solved among the plurality of problem items; and
a display configured to display the item to be solved and a solution situation graphic corresponding to the item to be solved.

13. The device of claim 12, wherein the problem solving table represents movement of a tooth for solving the plurality of problem items as at least one of a first direction movement corresponding to an occlusal direction, a second direction movement corresponding to a buccal direction, or a third direction movement corresponding to a mesial direction, and represents rotation of a tooth for solving the plurality of problem items as at least one of a first rotation about the first direction, a second rotation about the second direction, or a third rotation about the third direction.

14. The device of claim 13, wherein the problem solving table indicates, for each of a plurality of teeth, the movement or rotation of a tooth for solving the plurality of problem items, among the first direction movement, the second direction movement, the third direction movement, the first rotation, the second rotation, and the third rotation.

15. The device of claim 13, wherein the processor is configured to
determine, on the basis of the comparison result, at least one movement or rotation to be performed during orthodontic treatment from among the first direction movement, the second direction movement, the third direction movement, the first rotation, the second rotation, and the third rotation,
determine at least one problem item corresponding to the at least one determined movement or rotation based on the problem solving table, and
determine the at least one determined problem item as the item to be solved.

16. The device of claim 12, wherein the processor is configured to
determine an expected item to be solved, which is a problem item expected to require resolution among the plurality of problem items,
determine the item to be solved by updating the expected item to be solved based on user input, and
obtain the plurality of expected orthodontic models based on the item to be solved.

17. A computer-readable recording medium having recorded thereon a program which, when executed by a computer, performs the method of any one of claims 1 to 11.
